(19) Europäisches Patentamt
European Patent Office
Office européen des brevets

(11) EP 3 456 755 A1

(12) # EUROPÄISCHE PATENTANMELDUNG

(43) Veröffentlichungstag:
**20.03.2019 Patentblatt 2019/12**

(51) Int Cl.:
***C08G 18/79*** *(2006.01)* ***C07C 229/24*** *(2006.01)*
***C09D 175/02*** *(2006.01)* ***C08G 18/38*** *(2006.01)*

(21) Anmeldenummer: **17191792.5**

(22) Anmeldetag: **19.09.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude 4825**
**51365 Leverkusen (DE)**

(54) **POLYASPARAGINSÄUREESTER-ZUSAMMENSETZUNGEN ENTHALTEND POLYASPARAGINSÄUREESTER MIT PRIMÄREN AMINOGRUPPEN UND GERINGE MENGEN AN FUMARSÄUREDIALKYLESTER**

(57) Die vorliegende Erfindung betrifft Polyaspraginsäureester-Zusammensetzungen enthaltend Polyasparaginsäureester mit primären Aminogruppen und geringe Mengen an Fumarsäuredialkylester, ein Verfahren zu deren Herstellung und deren Einsatz als reaktive Komponente für Polyisocyanate in Zweikomponenten-Polyurethan-Systemen.

EP 3 456 755 A1

**Beschreibung**

Die vorliegende Erfindung betrifft Polyaspraginsäureester-Zusammensetzungen enthaltend Polyasparaginsäureester mit primären Aminogruppen und geringe Mengen an Fumarsäuredialkylester, ein Verfahren zu deren Herstellung und deren Einsatz als reaktive Komponente für Polyisocyanate in Zweikomponenten-Polyurethan-Systemen.

Zweikomponenten (2K)-Beschichtungsmittel, die als Bindemittel eine Polyisocyanatkomponente in Kombination mit einer gegenüber Isocyanatgruppen reaktionsfähigen Reaktivkomponente, insbesondere einer Polyhydroxylkomponente enthalten, sind seit langem bekannt. Sie eignen sich zur Herstellung von hochwertigen Überzügen, die hart, elastisch, abrieb- und lösemittelbeständig und vor allem auch witterungsstabil eingestellt werden können. Innerhalb dieser 2K-Polyurethan-Beschichtungstechnologie haben sich in letzter Zeit bestimmte, estergruppenhaltige, sekundäre Polyamine etabliert, die sich in Kombination mit Lackpolyisocyanaten insbesondere als Bindemittel in lösemittelarmen oder -freien (high solids) Beschichtungsmitteln eignen und eine rasche Aushärtung der Beschichtungen bei niedrigen Temperaturen ermöglichen.

Bei diesen sekundären Polyaminen handelt es sich um die sogenannten Polyasparaginsäureester, wie sie beispielhaft in der EP0403921 beschrieben werden. Ihre Verwendung allein oder in einem Gemisch mit weiteren, gegenüber Isocyanatgruppen reaktionsfähigen Komponenten in 2K-Polyurethan-Beschichtungsmitteln wird beispielsweise in der EP0403921, EP0639628, EP0667362, EP0689881, US5214086, EP0699696, EP0596360, EP0893458, DE19701835 und US5243012 beschrieben.

Die Synthese der Polyasparaginsäureester ist an sich bekannt und erfolgt über eine Addition von primären Polyaminen an eine aktivierte Kohlenstoff-Doppelbindung vinyloger Carbonylverbindungen, wie beispielsweise in Malein- oder Fumarsäureestern enthalten, die in der Literatur hinreichend beschrieben ist (Hauben Weyl, Meth. d. Org. Chemie Bd. 11/1, 272 (1957), Usp. Khim. 1969, 38, 1933). Bei dieser Reaktion kann es zur Bildung eines Polyasparaginsäureester mit primären Aminogruppen als Nebenprodukt kommen, wenn nur eine Aminogruppe des Polyamins mit der Doppelbindung der vinylogen Carbonylverbindungen reagiert hat. In den kommerziell erhältlichen Polyasparaginsäureestern wird Maleinsäureester als die vinyloge Carbonylverbindung eingesetzt. Während der Herstellung eines Polyasparaginsäureester auf Basis von Maleinsäureester kann eine retro-Michael Addition als eine weitere unerwünschte Nebenreaktion erfolgen, bei der durch Eliminierung des Polyamins als Nebenkomponente Fumarsäuredialkylester gebildet wird. Ein typisches Herstellverfahren eines Polyasparaginsäureesters erfordert daher eine Lagerzeit von 4-6 Wochen nachdem der Hauptteil der Edukte miteinander reagiert hat. In dieser Zeit erfolgt die sogenannte Reifung des Produktes, welche sich durch Stabilisierung der Viskosität manifestiert. Dadurch, dass der Umsatz innerhalb dieser Zeit weiter steigt, sinkt auch der Gehalt an Fumarsäuredialkylester. Diese Lagerung über mehrere Wochen führt zu erheblichen Logistik-Kosten innerhalb der Produktion. Obwohl das Produkt erst nach der Lagerung an den Kunden ausgeliefert wird, enthält es immer noch substantielle Mengen Fumarsäuredialkylester welches eine starke Sensibilisierung verursachen kann. Fumarsäurediethylester ist beispielsweise als VOC (volatile organic compounds) klassifiziert und verhindert damit eine Bereitstellung VOC-freier Beschichtungen. Ein weiterer Nachteil, der durch die Anwesenheit von Fumarsäuredialkylester hervorgerufen wird, ist die Erniedrigung der Glassübergangstemperatur eines Lackfilmes durch seine weichmachende Wirkung.

Aufgrund der oben beschriebenen Nachteile, die Polyasparaginsäureester-Zusammensetzungen und deren Herstellung bislang mit sich bringen, besteht seit Langem der Bedarf an Fumarsäuredialkylester-armen Polyasparaginsäureester-Zusammensetzungen, die über ein Verfahren hergestellt werden können, welches keine oder eine verkürzte Reifezeit benötigt.

Es bestehen theoretisch zwei Möglichkeiten das Verfahren zur Herstellung von Polyasparaginsäureester-Zusammensetzungen zu ändern, damit Polyasparaginsäureester-Zusammensetzungen entstehen, die die oben genannten Nachteile nicht aufweisen. Die Reaktionszeit kann verlängert oder die Reaktionstemperatur erhöht werden. Ersteres scheidet aus ökonomischen Gründen aus. Eine Erhöhung der Reaktionstemperatur auf z.B. 100°C oder sogar auf 80°C führt wiederum zu einer drastischen Gelbfärbung des Produktes.

EP0816326 offenbart ein Verfahren zur Beschleunigung der Addition des Polyamins an Dialkylmaleinat bzw. zum Reduzieren des Fumarsäuredialkylesters durch die Zugabe eines speziellen Katalysators. Da trotz des Einsatzes eines Katalysators die Notwendigkeit einer Lagerung nicht verhindert werden kann, führt dieser Lösungsansatz nicht zum endgültig zufriedenstellenden Ergebnis. In EP1197507 ist die Zugabe von Thiol-Verbindungen als Fänger für Fumarsäuredialkylester beschrieben. Dadurch, dass die Thiol-Verbindingen bekanntermaßen eine erhebliche Geruchsbelästigung verursachen ist auch diese Lösung nicht in der Praxis umsetztbar.

Eine theoretische Möglichkeit des destillativen Aufarbeitens ist z.B. in der EP0403921 erwähnt. Es handelt sich dabei um ein destillatives Entfernen von Fumarsäuredialkylester innerhalb eines Verfahrens, bei dem ein Überschuß von Dialkylmaleinat eingesetzt wird. Diese Offenbarung gibt keine Beispiele und auch kein Verfahren zur Destillation an. Da in diesem Verfahren ein Überschuss an Diethylmaleinat eingesetzt wird ist nicht zu erwarten, dass das aufgearbeitete Produkt deutliche Mengen an Polyasparaginsäureestern mit primären Aminogruppen aufweist. Dieses Verfahren hat sich nicht durchgesetzt, da die eingesetzte überschüssige Menge an Diethylmaleinat zu einer schlechten

Zeit-Raum-Ausbeute und viel Abfall führt, was ökonomisch nicht vertretbar ist.

DE102006002153 beschreibt ebenfalls ein Produkt welches unter Einsatz eines Überschusses Dialkylmaleinat und anschließende destillative Entfernung von Fumarsäuredialkylester hergestellt wird. Es handelt sich um einen Diasparaginsäureester, der frei von primären Aminogruppen ist.

Die Herstellung von aminofunktionellen Asparaginsäureestern ist im Prinzip bekannt. WO15130501 und WO15130502 offenbaren Polyasparaginsäureester-Zusammensetzungen, die zwischen 15 und 30% Asparaginsäureester mit primären Aminogruppen (gemessen als Flächen-% im Gas Chromatogram) aufweisen. In beiden Schriften wird jedoch kein Vorteil aufgrund eines erhöhten Gehaltes an Asparaginsäureester mit primären Aminogruppen erkannt und Polyasparaginsäureester-Zusammensetzungen mit akzeptabler Topfzeit konnten nur durch eine weitere Umsetzung mit bevorzugt cycloaliphatischen Polyisocyanaten erreicht werden. Es ist anzuhnemen, dass die so hergestellten Beschichtungsmittel aufgrund des herkömmlichen Herstellverfahren keinen reduzierten Gehalt an Fumarsäurediethylester aufweisen.

Im Rahmen der vorliegenden Erfindung können Polyasparaginsäureester-Zusammensetzungen erzeugt werden, die einen Gehalt von 1% bis 20% Polyasparaginsäureester mit primären Aminogruppen (gemessen als Flächen-% im Gas Chromatogramm) und einen verringerten Gehalt von 0.01 Gew.-Prozent bis 3 Gew.-Prozent an Fumarsäuredialkylester aufweisen und gleichzeitig die im Stand der Technik bekannten Nachteile der Polyasparaginsäureester-Zusammensetzungen überwinden. Diese erfindungsgemäßen Zusammensetzungen konnten über ein Verfahren mit und ohne Lagerungsprozess hergestellt werden.

Es wurde festgestellt, dass eine Dünnschichtdestillation einer nicht gereiften Polyasparaginsäureester-Zusammensetzung (d.h. direkt nach der Herstellung) zu einem Produkt führt, welches Mengen an Fumarsäuredialkylester unter 1% aufweist. Da der Reaktionsumsatz zum Zeitpunkt der Dünnschichtdestillation ca. 90% beträgt, enthält das Produkt im Vergleich zur herkömmlichen Polyasparaginsäureester-Zusammensetzung einen deutlich erhöhten Anteil an Polyasparaginsäureester mit primären Aminogruppen. Erwartungsgemäß könnte der hohe Anteil an Polyasparaginsäureestern mit primären Aminogruppen zu einer kürzeren Topfzeit führen. Überraschenderweise wurde jedoch gefunden, dass die Topfzeit der erfindungsgemäßen Polyasparaginsäureester-Zusammensetzungen sich nicht von der Topfzeit einer herkömmlich hergestellten Polyasparaginsäureester-Zusammensetzung unterscheidet. Darüber hinaus weisen die erfindungsgemäßen Polyasparaginsäureester-Zusammensetzungen eine beschleunigte Trocknung und eine verbesserte Kondenswasserbeständigkeit als weitere technische Vorteile auf. Das Verfahren der Dünnschichtdestillation lässt sich auch an gereiften Polyasparaginsäureester-Zusammensetzungen anwenden. In diesem Fall ist der Vorteil nicht das Entfallen der Reifung, aber die verringerte Menge an Fumarsäuredialkylester und eine schnellere Trocknung durch den erhöhten Anteil an Polyasparaginsäureester mit primärer Aminogruppe.

Gegenstand der vorliegenden Erfindung ist eine Zusammensetzung enthaltend einen oder mehrere Polyasparaginsäureester der allgemeinen Formel (I)

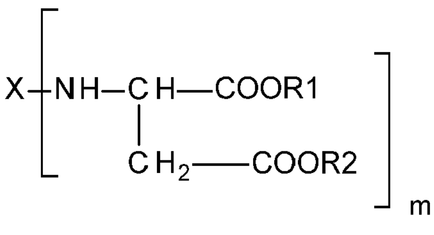

(I),

in welcher

| | |
|---|---|
| X | für einen m-wertigen, gegebenenfalls ein oder mehrere Heteroatome enthaltenden organischen Rest steht, wie er durch Entfernung der primären Aminogruppen aus einem entsprechenden, (cyclo)aliphatisch bzw. araliphatisch gebundene primäre Aminogruppen aufweisenden Polyamin des Molekulargewichtsbereichs 60 bis 6000 g/mol erhalten werden kann, und der weitere, gegenüber Isocyanatgruppen reaktive und/oder bei Temperaturen bis 100°C inerte funktionelle Gruppen enthalten kann, |
| R1 und R2 | für gleiche oder verschiedene organische Reste, mit je 1 bis 18 Kohlenstoffatomen stehen, |
| m | für eine ganze Zahl >1 steht, |

und

einen oder mehrere Polyasparaginsäureester mit primärer Aminogruppe der allgemeinen Formel (II)

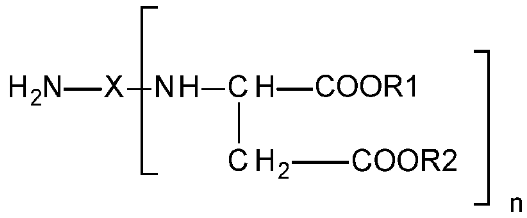

(II)

in welcher

n für m-1 steht,
X, Reste R1 und R2 die oben genannten Bedeutungen haben,

dadurch gekennzeichnet, dass der Anteil der Verbindung der allgemeinen Formel (II) von 1% bis 20% der GC Oberfläche (gemessen als Flächen-% im Gas Chromatogram), dem Anteil der beiden Verbindungen der allgemeinen Formel (I) und (II) entspricht, wobei die Summe der GC Oberflächen der beiden Verbindungen der allgemeinen Formel (I) und (II) 100% beträgt und ein Anteil an Fumarsäuredialkylester von 0.01 bis 3 Gew.-Prozent vorliegt.

Bevorzugt sind die erfindungsgemäßen Polyasparaginsäureester-Zusammensetzungen bei denen R1 und R2 für gleiche oder verschiedene Alkylreste mit je 1 bis 18 Kohlenstoffatomen, bevorzugt gleiche oder verschiedene Alkylreste mit je 1 bis 8 Kohlenstoffatomen und ganz besonders bevorzugt für jeweils Alkylreste wie Methyl-, Ethyl-, Propyl-, iso-Propyl-, Butyl- oder iso-Butyl-Reste stehen. Ganz besonders bevorzugt ist Ethyl.

Erfindungsgemäße Polyasparaginsäureester-Zusammensetzungen sind solche, bei denen X für organische Reste steht, die durch Entfernung der primären Aminogruppen aus einem entsprechenden, (cyclo)aliphatisch bzw. araliphatisch gebundene primäre Aminogruppen aufweisenden Polyamin erhalten werden, ausgewählt aus der folgenden Gruppe: alle bekannten Polyamine mit primären Aminogruppen, die der allgemeinen Formel (III) entsprechen. Beispielhaft seien die folgenden Verbindungen genannt: Ethylendiamin, 1,2-Diaminopropan, 1,4-Diaminobutan, 1,5-Diaminopentan, 2,5-Diamino-2,5-dimethylhexan, 1,5-Diamino-2-methylpentan (Dytek®A, Fa DuPont), 1,6-Diaminohexan, 2,2,4- und/oder 2,4,4-Trimethyl-1,6-diaminohexan, 1,11-Diaminoundecan, 1,12-Diaminododecan oder Triaminononan, Etheramine, wie z.B. 4,9-dioxadodecane-1,12-diamine, 4,7,10-trioxatridecane-1,13-diamine, oder höhermolekulare Polyetherpolyamine mit aliphatisch gebundenen, primären Aminogruppen, wie sie beispielsweise unter der Bezeichnung Jeffamin® von der Firma Huntsman vertrieben werden. Ebenfalls einsetzbar sind aliphatische polyzyklische Polyamine, wie Tricyclodecanbismethylamin (TCD-Diamin) oder Bis(aminomethyl)norbornane, amino-funktionelle Siloxane, z.B. Diaminopropylsiloxan G10 DAS (Fa. Momentive), Fettalkyl-basierte Amine, wie z.B. Fentamine der Fa. Solvay, Dimerfettsäurediamine wie z.B Priamine der Fa. Croda.

Bevorzugt sind die erfindungsgemäßen Polyasparaginsäureester-Zusammensetzungen bei denen X für organische Reste steht, die durch Entfernung der primären Aminogruppen aus einem der Polyamine der allgemeinen Formel (III) erhalten wird, worin m = 2 bedeutet und X für einen cyclischen Kohlenwasserstoffrest mit mindestens einem cyclischen Kohlenstoffring steht. Beispiele für besonders bevorzugt einsetzbare Diamine sind 1-Amino-3,3,5-trimethyl-5-aminomethylcyclohexan (IPDA), 2,4- und/oder 2,6-Hexahydrotoluylendiamin (H6-TDA), Isopropyl-2,4-diaminocyclohexan, und/oder Isopropyl-2,6-diaminocyclohexan, 1,3-Bis-(aminomethyl)-cyclohexan, 2,4'-, und/oder 4,4'-Diamino-dicyclohexylmethan, 3,3'-Dimethyl-4,4'-diamino-dicyclohexylmethan (Laromin® C 260, Fa. BASF AG), die isomere, eine Methylgruppe als Kernsubstituenten aufweisende Diaminodicyclohexylmethane (=C-Monomethyl-diaminodicyclohexylmethane), 3(4)-Aminomethyl-1-methylcyclohexylamin (AMCA) sowie araliphatische Diamine, wie z.B. 1,3-Bis-(aminomethyl)-benzol oder m-Xylylendiamin.

Ebenfalls bevorzugt sind die erfindungsgemäßen Polyasparaginsäureester-Zusammensetzungen bei denen X für organische Reste steht, die durch Entfernung der primären Aminogruppen aus einem der Polyamine der allgemeinen Formel (III) erhalten wird, ausgewählt aus der Gruppe: Polyetherpolyamine mit aliphatisch gebundenen primären Aminogruppen, 1,2 Diaminopropan, 1,4-Diaminobutan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 1,5-Diaminopentan, 2,4'- und/oder 4,4'-Diamino-dicyclohexylmethan oder 3,3'-Dimethyl-4,4'-diamino-dicyclohexylmethan. Besonders bevorzugt sind 3,3'-Dimethyl-4,4'-diamino-dicyclohexylmethan, 1,5-Diaminopentan, 2,4'- und/oder 4,4'-Diaminodicyclohexylmethan, 1,5-Diamino-2-methylpentan und ganz besonders bevorzugt ist die Verwendung von 2,4'- und/oder 4,4'-Diamino-dicyclohexylmethan.

Besonders bevorzugt sind die erfindungsgemäßen Polyasparaginsäureester-Zusammensetzungen bei denen X für organische Reste steht, die durch Entfernung der primären Aminogruppen aus einem der Polyamine der allgemeinen Formel (III) erhalten wird, ausgewählt aus der Gruppe: Polyetherpolyamine mit aliphatisch gebundenen primären Aminogruppen, 1,2 Diaminopropan, 1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 2,4'- und/oder 4,4'-Diamino-dicyclohexylmethan oder 3,3'-Dimethyl-4,4'diamino-dicyclohexylmethan.

Ganz besonders bevorzugt sind die erfindungsgemäßen Polyasparaginsäureester-Zusammensetzungen bei denen X für organische Reste steht, die durch Entfernung der primären Aminogruppen aus einem der Polyamine der allgemeinen Formel (III) erhalten wird, ausgewählt aus der Gruppe: 3,3'-Dimethyl-4,4'diaminodicyclohexylmethan, 2,4'- und/oder 4,4'-Diaminodicyclohexylmethan, 1,5-Diamino-2-methylpentan.

Vorzugsweise steht m für eine ganze Zahl >1 und bevorzugt für 2.

Bevorzugt sind die erfindungsgemäßen Polyasparaginsäureester-Zusammensetzungen enthaltend einen Anteil von 1% bis 20%, bevorzugt 4% bis 20%, besonders bevorzugt bis 4% bis 15% der GC Oberfläche (gemessen als Flächen-% im Gas Chromatogram) der beiden Verbindungen der allgemeinen Formel (I) und (II) entspricht, wobei die Summe der GC Oberflächen der beiden Verbindungen der allgemeinen Formel (I) und (II) 100% beträgt.

Bevorzugt sind die erfindungsgemäßen Polyasparaginsäureester-Zusammensetzungen enthaltend einen Anteil von 0.01 bis 3 Gew.-Prozent, bevorzugt 0.01 bis 1 Gew.-Prozent, besonders bevorzugt 0.01 bis 0.1 Gew.-Prozent an Fumarsäuredialkylester.

Besonders bevorzugter Gegenstand der vorliegenden Erfindung ist eine Zusammensetzung enthaltend einen oder mehrere Polyasparaginsäureester der allgemeinen Formel (I),
in welcher

| | |
|---|---|
| X | für einen m-wertigen, gegebenenfalls ein oder mehrere Heteroatome enthaltenden organischen Rest steht, wie er durch Entfernung von primären Aminogruppen aus Polyetherpolyaminen mit aliphatisch gebundenen primären Aminogruppen, 1,2 Diaminopropan, 1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimethyl-5-aminomethylcyclohexan, 2,4- und/oder 2,6-Hexahydrotoluylendiamin, 2,4'- und/oder 4,4'-Diaminodicyclohexylmethan oder 3,3'-Dimethyl-4,4'diamino-dicyclohexylmethan erhalten wird, |
| R1 und R2 | für gleiche oder verschiedene Alkylreste mit je 1 bis 8 Kohlenstoffatomen stehen, |
| m | für eine ganze Zahl > 1 steht, |

und
einen oder mehrere Polyasparaginsäureester mit primärer Aminogruppe der allgemeinen Formel (II),
in welcher

| | |
|---|---|
| n | für m-1 steht, |
| X, Reste R1 und R2 | die oben genannten Bedeutungen haben, |

dadurch gekennzeichnet, dass der Anteil der Verbindung der allgemeinen Formel (II) von 4% bis 20% der GC Oberfläche (gemessen als Flächen-% im Gas Chromatogram), dem Anteil der beiden Verbindungen der allgemeinen Formel (I) und (II) entspricht, wobei die Summe der GC Oberflächen der beiden Verbindungen der allgemeinen Formel (I) und (II) 100% beträgt und ein Anteil an Fumarsäuredialkylester von 0.01 bis 1 Gew.-Prozent vorliegt.

Ganz besonders bevorzugter Gegenstand der vorliegenden Erfindung ist eine Zusammensetzung enthaltend einen oder mehrere Polyasparaginsäureester der allgemeinen Formel (I),
in welcher

| | |
|---|---|
| X | für einen m-wertigen organischen Rest steht, wie er durch Entfernung von primären Aminogruppen aus 3,3'-Dimethyl-4,4'diaminodicyclohexylmethan, 2,4'- und/oder 4,4'-Diaminodicyclohexylmethan, 1,5-Diamino-2-methylpentan erhalten werden kann, |
| R1 und R2 | für gleiche oder verschiedene Alkylreste ausgewählt aus der Gruppe Methyl-, Ethyl-, Propyl-, iso-Propyl-, Butyl- oder iso-butyl-Reste stehen, |
| m | für 2 steht, |

und
einen oder mehrere Polyasparaginsäureester mit primärer Aminogruppe der allgemeinen Formel (II),
in welcher

| | |
|---|---|
| n | für m-1 steht, |
| X, Reste R1 und R2 | die oben genannten Bedeutungen haben, |

dadurch gekennzeichnet, dass der Anteil der Verbindung der allgemeinen Formel (II) von 4% bis 15% der GC Oberfläche (gemessen als Flächen-% im Gas Chromatogram), dem Anteil der beiden Verbindungen der allgemeinen Formel (I) und (II) entspricht, wobei die Summe der GC Oberflächen der beiden Verbindungen der allgemeinen Formel (I) und (II) 100% beträgt und ein Anteil an Fumarsäuredialkylester 0.01 bis 0.1 Gew.-Prozent vorliegt.

Ein weiterer besonders bevorzugter Gegenstand der vorliegenden Erfindung ist eine Zusammensetzung enthaltend einen oder mehrere Polyasparaginsäureester der allgemeinen Formel (I), in welcher

X für einen m-wertigen organischen Rest steht, wie er durch Entfernung von primären Aminogruppen aus 3,3'-Dimethyl-4,4'diaminodicyclohexylmethan, 2,4'- und/oder 4,4'-Diaminodicyclohexylmethan erhalten werden kann,

R1 und R2 für Ethyl-Reste stehen,

m für 2 steht,

und einen oder mehrere Polyasparaginsäureester mit primärer Aminogruppe der allgemeinen Formel (II), in welcher

n für m-1 steht,

X, Reste R1 und R2 die oben genannten Bedeutungen haben,

dadurch gekennzeichnet, dass der Anteil der Verbindung der allgemeinen Formel (II) von 4% bis 15% der GC Oberfläche (gemessen als Flächen-% im Gas Chromatogram), dem Anteil der beiden Verbindungen der allgemeinen Formel (I) und (II) entspricht, wobei die Summe der GC Oberflächen der beiden Verbindungen der allgemeinen Formel (I) und (II) 100% beträgt und ein Anteil an Fumarsäuredialkylester 0.01 bis 0.1 Gew.-Prozent vorliegt.

Bevorzugt sind erfindungsgemäßen Polyasparaginsäureester-Zusammensetzungen, die eine Platin-Cobalt-Farbzahl ≤ 100, besonders bevorzugt ≤ 50 aufweisen. Die Messung der Platin-Cobalt-Farbzahl erfolgt gemäß DIN EN ISO 6271:2016-05.

Weiterer Gegenstand der Erfindung ist ein Verfahren zur Herstellung der Zusammensetzung enthaltend ein oder mehrere Polyasparaginsäureester der allgemeinen Formel (I)

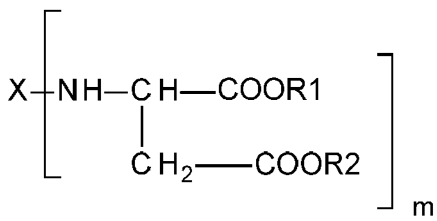

(I),

in welcher

X für einen m-wertigen, gegebenenfalls ein oder mehrere Heteroatome enthaltenden organischen Rest steht, wie er durch Entfernung der primären Aminogruppen aus einem entsprechenden, (cyclo)aliphatisch bzw. araliphatisch gebundene primäre Aminogruppen aufweisenden Polyamin des Molekulargewichtsbereichs 60 bis 6000 g/mol erhalten werden kann, und der weitere, gegenüber Isocyanatgruppen reaktive und/oder bei Temperaturen bis 100°C inerte funktionelle Gruppen enthalten kann,

R1 und R2 für gleiche oder verschiedene organische Reste, vorzugsweise für gleiche oder verschiedene Alkylreste mit je 1 bis 18 Kohlenstoffatomen und ganz besonders bevorzugt für gleiche oder verschiedene Alkylreste mit je 1 bis 8 Kohlenstoffatomen stehen,

m für eine ganze Zahl > 1 steht,

und einen oder mehrere Polyasparaginsäureester mit primärer Aminogruppe der allgemeinen Formel (II)

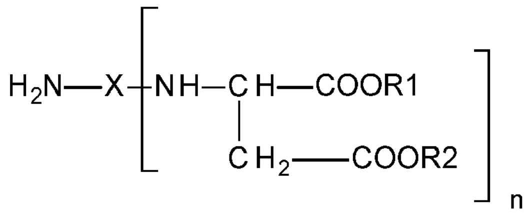

(II)

in welcher

| | |
|---|---|
| n | für m-1 steht, |
| X, Reste R1 und R2 | die oben genannten Bedeutungen haben, |

hergestellt durch Umsetzung von Polyaminen der allgemeinen Formel (III),

$X[NH_2]_m$ (III),

wobei X und m die oben genannte Bedeutung haben,
mit Verbindungen der allgemeinen Formel (IV)

R1OOC-CH=CH-COOR2 (IV),

wobei die Reste R1 und R2 die oben genannte Bedeutung haben,
dadurch gekennzeichnet, dass die daraus resultierende Polyasparaginsäureester-Zusammensetzung einen Anteil der Verbindung der allgemeinen Formel (II) von 1% bis 20% der GC Oberfläche (gemessen als Flächen-% im Gas Chromatogram), der dem Anteil beiden Verbindungen der allgemeinen Formel (I) und (II) entspricht, wobei die Summe der GC Oberflächen der beiden Verbindungen der allgemeinen Formel (I) und (II) 100% beträgt und einen Anteil an Fumarsäuredialkylester von 0.01 bis 3 Gew.-Prozent enthält.

Weiterer bevorzugter Gegenstand der vorliegenden Erfindung ist das oben offenbarte Verfahren zur Herstellung der Zusammensetzung enthaltend ein oder mehrere Polyasparaginsäureester der allgemeinen Formel (I), in welcher

| | |
|---|---|
| X | für einen m-wertigen, gegebenenfalls ein oder mehrere Heteroatome enthaltenden organischen Rest steht, wie er durch Entfernung von primären Aminogruppen aus Polyetherpolyaminen mit aliphatisch gebundenen primären Aminogruppen, 1,2 Diaminopropan, 1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 2,4'- und/oder 4,4'-Diaminodicyclohexylmethan oder 3,3'-Dimethyl-4,4'diamino-dicyclohexylmethan erhalten wird, |
| R1 und R2 | für gleiche oder verschiedene Alkylreste mit je 1 bis 8 Kohlenstoffatomen stehen, |
| m | für eine ganze Zahl >1 steht, |

und
einen oder mehrere Polyasparaginsäureester mit primärer Aminogruppe der allgemeinen Formel (II), in welcher

| | |
|---|---|
| n | für m-1 steht, |
| X, Reste R1 und R2 | die oben genannten Bedeutungen haben, |

hergestellt durch Umsetzung von Polyaminen der allgemeinen Formel (III), wobei X und m die oben genannte Bedeutung haben, mit Verbindungen der allgemeinen Formel (IV), wobei die Reste R1 und R2 die oben genannte Bedeutung haben, dadurch gekennzeichnet, dass die daraus resultierende Polyasparaginsäureester-Zusammensetzung einen Anteil der Verbindung der allgemeinen Formel (II) von 4% bis 20% der GC Oberfläche (gemessen als Flächen-% im Gas Chromatogram), der dem Anteil derbeiden Verbindungen der allgemeinen Formel (I) und (II) entspricht, wobei die Summe der GC Oberflächen der beiden Verbindungen der allgemeinen Formel (I) und (II) 100% beträgt und einen Anteil an Fumarsäuredialkylester von 0.01 bis 1 Gew.-Prozent enthält.

Weiterer bevorzugter Gegenstand der vorliegenden Erfindung ist das oben offenbarte Verfahren zur Herstellung der Zusammensetzung enthaltend ein oder mehrere Polyasparaginsäureester der allgemeinen Formel (I), in welcher

| | |
|---|---|
| X | für einen m-wertigen organischen Rest steht, wie er durch Entfernung von primären Aminogruppen aus 3,3'-Dimethyl-4,4'diaminodicyclohexylmethan, 2,4'- und/oder 4,4'-Diamino-dicyclohexylmethan, 1,5-Diamino-2-methylpentan erhalten werden kann, |
| R1 und R2 | für gleiche oder verschiedene Alkylreste ausgewählt aus der Gruppe Methyl-, Ethyl-, Propyl-, iso-Propyl-, Butyl- oder iso-Butyl-Reste stehen, |
| m | für 2 steht, |

und
einen oder mehrere Polyasparaginsäureester mit primärer Aminogruppe der allgemeinen Formel (II), in welcher

| | |
|---|---|
| n | für m-1 steht, |
| X, Reste R1 und R2 | die oben genannten Bedeutungen haben, |

hergestellt durch Umsetzung von Polyaminen der allgemeinen Formel (III), wobei X und m die oben genannte Bedeutung haben, mit Verbindungen der allgemeinen Formel (IV), wobei die Reste R1 und R2 die oben genannte Bedeutung haben, dadurch gekennzeichnet, dass die daraus resultierende Polyasparaginsäureester-Zusammensetzung einen Anteil der Verbindung der allgemeinen Formel (II) von 4% bis 15% der GC Oberfläche (gemessen als Flächen-% im Gas Chromatogram), der dem Anteil der beiden Verbindungen der allgemeinen Formel (I) und (II) entspricht, wobei die Summe der GC Oberflächen der beiden Verbindungen der allgemeinen Formel (I) und (II) 100% beträgt und einen Anteil an Fumarsäuredialkylester 0.01 bis 0.1 Gew.-Prozent enthält.

Weiterer bevorzugter Gegenstand der vorliegenden Erfindung ist das oben offenbarte Verfahren zur Herstellung der Zusammensetzung enthaltend ein oder mehrere Polyasparaginsäureester der allgemeinen Formel (I), in welcher

| | |
|---|---|
| X | für einen m-wertigen organischen Rest steht, wie er durch Entfernung von primären Aminogruppen aus 2,4-und/oder 4,4'-Diaminodicyclohexylmethan erhalten werden kann, |
| R1 und R2 | für Ethyl-Reste stehen, |
| m | für 2 steht, |

und
einen oder mehrere Polyasparaginsäureester mit primärer Aminogruppe der allgemeinen Formel (II),
in welcher

| | |
|---|---|
| n | für m-1 steht, |
| X, Reste R1 und R2 | die oben genannten Bedeutungen haben, |

hergestellt durch Umsetzung von Polyaminen der allgemeinen Formel (III), wobei X und m die oben genannte Bedeutung haben, mit Verbindungen der allgemeinen Formel (IV), wobei die Reste R1 und R2 die oben genannte Bedeutung haben, dadurch gekennzeichnet, dass die daraus resultierende Polyasparaginsäureester-Zusammensetzung einen Anteil der Verbindung der allgemeinen Formel (II) von 4% bis 15% der GC Oberfläche (gemessen als Flächen-% im Gas Chromatogram), der dem Anteil der beiden Verbindungen der allgemeinen Formel (I) und (II) entspricht, wobei die Summe der GC Oberflächen der beiden Verbindungen der allgemeinen Formel (I) und (II) 100% beträgt und einen Anteil an Fumarsäuredialkylester 0.01 bis 0.1 Gew.-Prozent enthält.

Vorzugsweise erfolgt das erfindungsgemäße Verfahren zur Herstellung der Zusammensetzung enthaltend Polyasparaginsäureester der allgemeinen Formel (I) und ein oder mehrere Polyasparaginsäureester mit primären Aminogruppen der allgemeinen Formel (II) in zwei Schritten. Im ersten Schritt die Verbindungen der allgemeinen Formel (III) und (IV) bei Temperaturen zwischen 0°C und 100°C, vorzugsweise 20 bis 80°C und besonders bevorzugt 20 bis 60°C, in einem Verhältnis der Äquivalente der primären Aminogruppen der Verbindungen der allgemeinen Formel (III) zu den C=C-Doppelbindungsäquivalenten der Verbindungen der allgemeinen Formel (IV) von 1:1.2 bis 1.2:1, vorzugsweise jedoch 1:1.05 bis 1.05:1 bis zu einem Restgehalt an Verbindungen der allgemeinen Formel (IV) von 2 bis 15 Gew.-Prozent, bevorzugt von 3 bis 10 Gew.-Prozent umgesetzt.

Im zweiten Schritt wird der nicht umgesetzte Anteil an der Verbindungen der allgemeinen Formel (IV) destillativ entfernt.

Geeignete Bedingungen während der Destillation sind ein Druckbereich zwischen 0.01 und 2 mbar und eine Temperatur des Sumpfablaufs beim Austritt aus der Destillationsapparatur ≤ 170 °C und ≥ der Temperatur liegt, die sich aus der folgenden Formel (V) ergibt:

$$T(\text{Sumpfablauf}) = 27 \times \ln(p) + 150 \quad (V)$$

wobei

| | |
|---|---|
| T(Sumpfablauf) | für die Temperatur des Sumpfablaufs in °C und |
| p | für den Druck in der Destillationsapparatur in mbar stehen. |

Durch Einhalten dieses Druckbereichs wird gewährleistet, dass einerseits moderate Temperaturen im Sumpfablauf ausreichen, um die Fumarsäuredialkylester im gewünschten Umfang abzureichern, andererseits das Verfahren im technischen Maßstab anwendbar bleibt. Bei geringerem Druck wird die Gasdichte zu gering und die erforderlichen Apparate deshalb so groß, dass das Verfahren aus wirtschaftlicher Sicht nachteilig wird.

Bevorzugt ist die Temperatur des Sumpfablaufs ≤ 170 °C, dabei jedoch mindestens 20 K oberhalb der Temperatur, die sich aus der Formel (V) ergibt, besonders bevorzugt liegt sie zwischen 20 K und 40 K oberhalb der Temperatur, die sich aus der Formel (V) ergibt jedoch nicht oberhalb von 170 °C.

Verbindungen der allgemeinen Formel (III), die in dem erfindungsgemäßen Verfahren Anwendung finden sind alle bekannten Polyamine mit primären Aminogruppen, die der allgemeinen Formel (III) entsprechen. Beispielhaft seien die folgenden Verbindungen genannt: Ethylendiamin, 1,2-Diaminopropan, 1,4-Diaminobutan, 1,5-Diaminopentan, 2,5-Diamino-2,5-dimethylhexan, 1,5-Diamino-2-methylpentan (Dytek®A, Fa DuPont), 1,6-Diaminohexan, 2,2,4- und/oder 2,4,4-Trimethyl-1,6-diaminohexan, 1,11-Diaminoundecan,1,12-Diaminododecan oder Triaminononan, Etheramine, wie z.B. 4,9-dioxadodecane-1,12-diamine, 4,7,10-trioxatridecane-1,13-diamine, oder höhermolekulare Polyetherpolyamine mit aliphatisch gebundenen, primären Aminogruppen, wie sie beispielsweise unter der Bezeichnung Jeffamin® von der Firma Huntsman vertrieben werden. Ebenfalls einsetzbar sind aliphatische polyzyklische Polyamine, wie Tricyclodecanbismethylamin (TCD-Diamin) oder Bis(aminomethyl)norbornane, amino-funktionelle Siloxane, z.B. Diaminopropylsiloxan G10 DAS (Fa. Momentive), Fettalkyl-basierte Amine, wie z.B. Fentamine der Fa. Solvay, Dimerfettsäurediamine wie z.B Priamine der Fa. Croda.

Bevorzugt werden im erfindungsgemäßen Verfahren Polyamine der allgemeinen Formel (III) eingesetzt worin m = 2 bedeutet und X für einen cyclischen Kohlenwasserstoffrest mit mindestens einem cyclischen Kohlenstoffring steht. Beispiele für besonders bevorzugt einsetzbare Diamine sind 1-Amino-3,3,5-trimethyl-5-aminomethylcyclohexan (IPDA), 2,4- und/oder 2,6-Hexahydrotoluylendiamin (H6-TDA), Isopropyl-2,4-diaminocyclohexan, und/oder Isopropyl-2,6-diaminocyclohexan, 1,3-Bis-(aminomethyl)-cyclohexan, 2,4'- und/oder 4,4'-Diaminodicyclohexylmethan, 3,3'-Dimethyl-4,4'-diamino-dicyclohexylmethan (Laromin® C 260, Fa. BASF AG), die isomere, eine Methylgruppe als Kernsubstituenten aufweisende Diaminodicyclohexylmethane (=C-Monomethyl-diaminodicyclohexylmethane), 3(4)-Aminomethyl-l-methylcyclohexylamin (AMCA) sowie araliphatische Diamine, wie z.B. 1,3-Bis-(aminomethyl)-benzol oder m-Xylylendiamin.
Ebenfalls bevorzugt werden im erfindungsgemäßen Verfahren Polyamine der allgemeinen Formel (III) eingesetzt ausgewählt aus der Gruppe: Polyetherpolyamine mit aliphatisch gebundenen primären Aminogruppen, 1,2 Diaminopropan, 1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 2,4'- und/oder 4,4'-Diamino-dicyclohexylmethan oder 3,3'-Dimethyl-4,4'-diamino-dicyclohexylmethan. Besonders bevorzugt sind 3,3'-Dimethyl-4,4'-diamino-dicyclohexylmethan, 2,4'- und/oder 4,4'-Diaminodicyclohexylmethan, 1,5-Diamino-2-methylpentan und ganz besonders bevorzugt ist die Verwendung von 2,4- und/oder 4,4'-Diaminodicyclohexylmethan.
Besonders bevorzugt sind die erfindungsgemäßen Polyasparaginsäureester-Zusammensetzungen bei denen X für organische Reste steht, die durch Entfernung der primären Aminogruppen aus einem der Polyamine der allgemeinen Formel (III) erhalten wird, ausgewählt aus der Gruppe: Polyetherpolyamine mit aliphatisch gebundenen primären Aminogruppen, 1,2 Diaminopropan, 1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 2,4'- und/oder 4,4'-Diamino-dicyclohexylmethan oder 3,3'-Dimethyl-4,4'diamino-dicyclohexylmethan.
Ganz besonders bevorzugt sind die erfindungsgemäßen Polyasparaginsäureester-Zusammensetzungen bei denen X für organische Reste steht, die durch Entfernung der primären Aminogruppen aus einem der Polyamine der allgemeinen Formel (III) erhalten wird, ausgewählt aus der Gruppe: 3,3'-Dimethyl-4,4'diaminodicyclohexylmethan, 2,4'- und/oder 4,4'-Diaminodicyclohexylmethan, 1,5-Diamino-2-methylpentan.

Bevorzugte Verbindungen der allgemeinen Formel (IV), die in dem erfindungsgemäßen Verfahren Anwendung finden sind Malein- oder Fumarsäureester der allgemeinen Formel (IV), worin R1 und R2 für gleiche oder verschiedene organische Reste mit je 1 bis 18 Kohlenstoffatomen stehen. Bevorzugt stehen R1 und R2 unabhängig voneinander für lineare oder verzweigte Alkylreste mit 1 bis 8 Kohlenstoffatomen. Beispielhaft für Verbindungen der allgemeinen Formel (IV) seien die folgenden Verbindungen genannt: Maleinsäuredimethylester, -diethylester, -di-n oder -isopropylester, -di-n-butylester, -di-2-ethyl-hexylester oder die entsprechenden Fumarsäureester. Besonders bevorzugt ist Maleinsäurediethylester.

Die erfindungsgemäßen Polyasparaginsäureester-Zusammensetzungen stellen wertvolle Reaktionspartner für

Polyisocyanate in lösemittelarmen oder -freien Zweikomponenten-Polyurethan-Systemen dar.
Gegenstand der Erfindung ist somit auch die Verwendung der erfindungsgemäßen Polyasparaginsäureester-Zusammensetzungen als Reaktivkomponente in Zweikomponenten-Polyurethan-Systemen oder zur Herstellung von Prepolymeren. Die Zweikomponenten(2K)-Polyurethan-Systeme, enthaltend die erfindungsgemäßen Polyasparaginsäureester-Zusammensetzungen, können dann als Beschichtungsmitteln zur Herstellung von Überzügen verwendet werden.
In den erfindungswesentlichen Beschichtungsmitteln können zusätzlich zu den erfindungswesentlichen Komponenten auch die in der Beschichtungstechnologie üblichen Hilfsstoffe, wie anorganische oder organische Pigmente, weitere organische Lichtschutzmittel, Radikalfänger, Lackadditive, wie Dispergier-, Verlauf-, Verdickungs-, Entschäumungs- und andere Hilfsmittel, Haftmittel, Fungizide, Bakterizide, Stabilisatoren oder Inhibitoren und Katalysatoren mit verwendet werden.

Die erfindungsgemäßen Beschichtungsmittel kommen vorzugsweise in den Bereichen Autoerstlackierung, Autoreparaturlackierung, Großfahrzeuglackierung, Kunststofflackierung, allgemeine Industrielackierung, Bodenbeschichtung und/oder Holz-/Möbellackierung zur Anwendung.

Ein weiterer Gegenstand der Erfindung sind daher auch beschichtete Substrate, die unter Einsatz der erfindungsgemäßen Polyasparaginsäureester-Zusammensetzungen erhältlich sind.

## Experimenteller Teil

### Rohstoffe:

PACM 20: ein Gemisch aus 2,4- und 4,4'-Diaminodicyclohexylmethan, Hersteller Fa. Evonik Desmodur N 3600: ein niedrigviskose HDI-Trimerisat mit ca. 23% NCO und ≤ 0,25% freies HDI, Hersteller Fa. Covestro
Desmodur N 3900: ein niedrigviskose HDI-Trimerisat mit ca. 23,5% NCO und ≤ 0,25% freies HDI, Hersteller Fa. Covestro
Byk 331: Polyether modifiziert Polydimethylsiloxan Oberflächenadditiv, Hersteller Fa. BYK Tinuvin 292: eine Mischung aus Bis(1,2,2,6,6-pentamethyl-4-piperidyl) sebacat und Methyl 1,2,2,6,6-pentamethyl-4-piperidyl sebacat, ein Lichtstabilisator von der Fa. BASF
Tinuvin 384-2: Benzenpropansäure, 3-(2H-benztriazol-2-yl)-5-(1,1-dimethylethyl)-4-hydroxy-, C7-9-verzweigt und linear Alkylester, ein Lichtstabilisator von der Fa. BASF

### Methoden:

Fumarsäurediethylester-Gehalte wurden quantitativ mit Hilfe einer GC-Methode mit einem internem Standard bestimmt. Es wurde ein 6890 Gaschromatograph der Fa. Agilent mit einer Standard-GC-Kapillare (100% Polysiloxan-Phase) und einem FID Detektor verwendet. Die Temperatur des Injektors (Splitausgang) war 180°C, als Trägergas wurde Helium verwendet. Die Bestimmungsgrenze diese Methode betrug 300 ppm.
GC-MS Messungen wurden mit einem 6890 Gaschromatograph und Massen Spektrum Detektor 5973 der Fa. Agilent mit Standard-Ionisierung (electron impact) mit 70eV, eine Standard-GC-Kapillare (100% Polysiloxan-Phase) und Split-Injektion bei 250°C Injektortemperatur durchgeführt. Ausgewertet wurden die % Flächen des Gaschromatograms.
Sämtliche Viskositatsmessungen erfolgten mit einem Physica MCR 51 Rheometer der Fa. Anton Paar Germany GmbH (DE) nach DIN EN ISO 3219:1994-10.
Die Bestimmung der NCO-Gehalte erfolgte titrimetrisch nach DIN EN ISO 11909:2007-05.
Die Hazen-Farbzahlen wurden an einem LICO 400 Farbmessgerät der Fa. Hach Lange GmbH, Düsseldorf, gemäß DIN EN ISO 6271:2016-05 ermittelt.
Die Bestimmung der Aminzahlen erfolgte titrimetrisch nach EN ISO 9702:1998 (Perchlorsäre-Methode) mit der Ausnahme, dass die Ergebnisse als Aminzahl angegeben wurden. Die Aminzahl in mg KOH/g wurde nach folgender Gleichung berechnet:

$$Aminzahl = \frac{(a - b)x\ 5{,}61}{E}$$

a: der Verbrauch, in Milliliter, an Perchlorsäure, c = 0,1 mol/l, im Hauptversuch;
b: der Verbrauch, in Milliliter, an Perchlorsäure, c = 0,1 mol/l, im Blindversuch;
E: die Einwaage, in Gramm.

Die Bestimmung der Auslaufzeiten erfolgte nach DIN EN ISO 2431:2012-03, mit der Ausnahme, dass ein DIN 4 Auslaufbecher verwendet wurde. Die Topfzeit wurde als die Zeit zur Verdopplung der Auslaufzeit definiert.
Die Bestimmung der Trockung erfolgte nach DIN EN ISO 9117-5:2012-11.

Zur Reaktivitätsbestimmung wurde folgende Gelzeitmessmethode entwickelt: Die Einsatzstoffe (Gesamtmenge 10g) wurden in einen Becher eingewogen und 15 Sekunden bei 3000 Upm in einem Speedmixer gemischt. Dann wurde eine gebogene Büroklammer oder eine Einwegpipette eingesetzt und mit einer Stoppuhr wurde die Zeit bestimmt bis das Gemisch Fäden zieht bzw. fest wird.

**Beispiel 1**

Kommerziell erhältlicher Polyasparaginsäureester der Fa. Covestro unter dem Namen Desmophen NH 1420.

| Kenndaten: | |
|---|---|
| Monoamine der Formel (II) (GC-MS): | 4.0% |
| Fumarsäurediethylester (GC) | 2.9 Gew.-Prozent |
| Viskosität | 1220 mPas |
| Farbzahl | 27 APHA |
| Aminzahl | 201 mg KOH/g |

**Beispiel 2**

341.8g PACM 20 wurden bei 23 °C unter trockenem Stickstoff und Rühren vorgelegt. Dazu wurden 839.4g Maleinsäurediethylester so zugetropft, dass die Temperatur nicht über 60 °C gestiegen ist. Nach der beendeten Zugabe wurde die Temperatur auf 45 °C eingestellt und der Ansatz wurde bei 45 °C eine Stunde gerührt. Anschließend wurde der Ansatz bei 23°C 8 Wochen gelagert. Der Gehalt an Fumarsäurediethylester betrug nach der Lagerung 0.04 Gew.-Prozent. Man erhielt ein farbhelles Produkt mit folgenden Kenndaten:

| | |
|---|---|
| Monoamine der Formel (II) (GC-MS): | 65.8% |
| Fumarsäurediethylester (GC) | 0.04 Gew.-Prozent |
| Viskosität | 690 mPas |
| Farbzahl | 16 APHA |
| Aminzahl | 293 mg KOH/g |

**Beispiel 3**

341.8g PACM 20 wurden bei 23 °C unter trockenem Stickstoff und Rühren vorgelegt. Dazu wurden 1678.8g Maleinsäurediethylester so zugetropft, dass die Temperatur nicht über 60 °C gestiegen ist. Nach der beendeten Zugabe wurde die Temperatur auf 45 °C eingestellt und der Ansatz wurde bei 45 °C eine Stunde gerührt. Anschließend wurde der Ansatz bei 23°C 24 Stunden gelagert. Der Gehalt an Fumarsäurediethylester betrug nach der Lagerung 11.5 Gew.-Prozent. Fumarsäurediethylester wurde dann bei 120 °C und 0.2 mbar abdestilliert. Man erhielt ein farbhelles Produkt mit folgenden Kenndaten:

| | |
|---|---|
| Monoamine der Formel (II) (GC-MS): | 25.4% |
| Fumarsäurediethylester (GC) | 0.05 Gew.-Prozent |
| Viskosität | 1330 mPas |
| Farbzahl | 8 APHA |
| Aminzahl | 224 mg KOH/g |

**Beispiel 4**

341.8g PACM 20 wurden bei 23 °C unter trockenem Stickstoff und Rühren vorgelegt. Dazu wurden 1678.8g Maleinsäurediethylester so zugetropft, dass die Temperatur nicht über 60 °C gestiegen ist. Nach der beendeten Zugabe wurde die Temperatur auf 45 °C eingestellt und der Ansatz wurde bei 45 °C 2 Stunden gerührt. Anschließend wurde der Ansatz bei 23 °C 7 Wochen gelagert. Der Gehalt an Fumarsäurediethylester betrug nach der Lagerung 2.7 Gew.-Prozent. Fumarsäurediethylester wurde dann bei 120 °C und 0.2 mbar abdestilliert. Man erhielt ein farbhelles Produkt mit folgenden Kenndaten:

| | |
|---|---|
| Monoamine der Formel (II) (GC-MS): | 5.3% |

(fortgesetzt)

| | |
|---|---|
| Fumarsäurediethylester (GC) | 0.08 Gew.-Prozent |
| Viskosität | 1810 mPas |
| Farbzahl | 19 APHA |
| Aminzahl | 203 mg KOH/g |

**Beispiel 5**

341.8g PACM 20 wurden bei 23 °C unter trockenem Stickstoff und Rühren vorgelegt. Dazu wurden 1678.8g Maleinsäurediethylester so zugetropft, dass die Temperatur nicht über 60 °C gestiegen ist. Nach der beendeten Zugabe wurde die Temperatur auf 45 °C eingestellt und der Ansatz wurde bei 45 °C zwei Stunden gerührt. Anschließend wurde der Ansatz bei 23°C 30 Stunden gelagert. Der Gehalt an betrug nach der Lagerung 8.62 Gew.-Prozent. Fumarsäurediethylester wurde dann bei 120 °C und 0.2 mbar abdestilliert. Man erhielt ein farbhelles Produkt mit folgenden Kenndaten:

| | |
|---|---|
| Monoamine der Formel (II) (GC-MS): | 13.2% |
| Fumarsäurediethylester (GC) | < 0.03 Gew.-Prozent |
| Viskosität | 1650 mPas |
| Farbzahl | 5 APHA |
| Aminzahl | 213 mg KOH/g |

**Beispiel 6**

341.8g PACM 20 wurden bei 23 °C unter trockenem Stickstoff und Rühren vorgelegt. Dazu wurden 1678.8g Maleinsäurediethylester so zugetropft, dass die Temperatur nicht über 60 °C gestiegen ist. Nach der beendeten Zugabe wurde die Temperatur auf 45 °C eingestellt und der Ansatz wurde bei 45 °C eine Stunde gerührt. Anschließend wurde der Ansatz bei 23°C 24 Stunden gelagert. Der Gehalt an Fumarsäurediethylester betrug nach der Lagerung 8.85 Gew.-Prozent. Fumarsäurediethylester wurde dann bei 120 °C und 0.2 mbar abdestilliert. Man erhielt ein farbhelles Produkt mit folgenden Kenndaten:

| | |
|---|---|
| Monoamine der Formel (II) (GC-MS) | 14.1% |
| Fumarsäurediethylester (GC) | 0.08 Gew.-Prozent |
| Viskosität | 1630 mPas |
| Farbzahl | 5 APHA |
| Aminzahl | 214 mg KOH/g |

Vor den anwendungstechnischen Ausprüfungen wurde die Reaktivität von ausgewählten Polyasparaginsäureestern mit der oben beschriebenen Gelzeitmessmethode bestimmt.

**Tabelle 1. Reaktivitätsbestimmung mittels** Gelzeitmessmethode

| Einsatzstoff | Desmodur N 3600 | Desmodur N 3600 | Desmodur N 3600 | Desmodur N 3600 | Desmodur N 3600 |
|---|---|---|---|---|---|
| Einwaage | 4.18 | 4.88 | 4.22 | 4.18 | 4.18 |
| Einsatzstoff | Beispiel 1 | Beispiel 2 | Beispiel 3 | Beispiel 5 | Beispiel 6 |
| Einwaage | 5.82 | 5.11 | 5.78 | 5.82 | 5.82 |
| Zeit bis: | | | | | |
| die Probe Fäden zieht | 37 min | n.g* | 5 min | 49 min | 52 min |
| die Probe ist fest | 65 min | n.g.* | 7 min | 88 min | 92 min |
| *Probe im Speedmixer geliert | | | | | |

Aus der Tabelle 1 ist es ersichtlich, dass die erfindungsgemäße Zusammensetzung enthaltend Polyasparaginsäureester aus dem Beispiel 5 und 6 trotz eines höheren Gehaltes an Polyasparaginsäureester mit primären Aminogruppen im Vergleich zu dem kommerziell erhältlichen Produkt keine verkürzte Gelzeit zeigen und sind somit für Lacke geeignet.

**Prüfung in Lacken**

Erfindungsgemäße Polyasparaginsäureester-Zusammensetzungen aus dem Beispiel 1 und 5 wurden in Lackformulierungen getestet.

Zubereitung eines Stammlackes

Zu der in der Tabelle 2 gegebenen Menge der Komponente C wurden die Additive sowie die in der Tabelle gegebene Menge Butylacetat gegeben und innig verrührt.

Zubereitung der Härterlösung

Zu der in der Tabelle 2 gegebenen Menge der Komponente D wurde die gegebene Menge Butylacetat gegeben und innig verrührt.

Vermischung des Stammlackes mit dem Härter und Applikation:

Der oben aufgeführter Stammlack und der Härter wurden jeweils zusammengemischt und innig verrührt. Anschließend wurden die Mischungen jeweils mit einer Luftpistole auf mit schwarzem Basislack vorbeschichtete Coil Coat Bleche appliziert, 10 min bei Raumtemperatur abgelüftet und anschließend bei Raumtemperatur und bei 60°C getrocknet. Es wurden brillante, hochglänzende Beschichtungen mit einer Schichtdicke von 50 µm erhalten.

Eine Übersicht über die ermittelten lacktechnischen Eigenschaften der Beschichtungen ist in Tabelle 3 dargestellt.

**Tabelle 2. Zusammensetzung der Stammlacke**

| Temperatur: 24°C | Beispiel 7 Erfindungsgemäß | Beispiel 8 erfindungsgemäß | Beispiel 9 Vergleich |
|---|---|---|---|
| Luftfeuchte: 48% | | | |
| Komponente C | | | |
| Beispiel 5 | 43.77 | | |
| Beispiel 4 | | 45.25 | |
| Beispiel 1 | | | 47.24 |
| Byk 331 (10%ig in BA) | 0.08 | 0.08 | 0.08 |
| Tinuvin 292 (50%ig in BA) | 0.16 | 0.17 | 0.16 |
| Tinuvin 384 - 2 (50%ig in BA) | 0.33 | 0.33 | 0.33 |
| Butylacetat | 21.34 | 20.00 | 16.19 |
| Komponente D | | | |
| Desmodur N 3900 (100%ig) | 29.75 | 29.42 | 30.65 |
| Butylacetat | 1.57 | 1.75 | 2.35 |

**Tabelle 3. Lacktechnische Eigenschaften von Beschichtungen**

| Temperatur: 24°C | | Beispiel 7 | Beispiel 8 | Beispiel 9 |
|---|---|---|---|---|
| Luftfeuchte: 48% | | | | |
| Festkörper in % bei Spritzviskosität (ber.) | | 73.4 | 74.9 | 81.1 |

(fortgesetzt)

| Temperatur: 24°C | | Beispiel 7 | Beispiel 8 | Beispiel 9 |
|---|---|---|---|---|
| Auslaufzeit DIN 4mm (sec.) | 0h | 17 | 16 | 17 |
| | 5’ | 18 | 17 | 18 |
| | 10’ | 19 | 18 | 20 |
| | 15’ | 21 | 19 | 24 |
| | 20’ | 23 | 20 | 27 |
| | 25’ | 27 | 22 | 31 |
| | 30’ | 32 | 25 | 40 |
| Trocknung (min.) | T 1 | 13 | 12 | 15 |
| RT | T 3 | 27 | 25 | 30 |
| | T 4 | 40 | 40 | 45 |
| Schichtdicke (µm) | | ca. 50 | ca. 50 | ca. 50 |
| Trocknung (min.) | T 1 | Sofort | sofort | sofort |
| 30’ - 60°C | T 3 | 15 | 15 | 20 |
| | T 4 | 27 | 25 | 30 |
| Schichtdicke (µm) | | ca. 50 | ca. 50 | ca. 50 |

Durch den Vergleich der in Tabelle 3 angegebenen lacktechnischen Eigenschaften von der Vergleich - Beschichtung (Beispiel 9) mit denen der erfindungsgemäßen Beschichtungen (Beispiele 7 und 8) lässt sich eine schnellere Trocknung bei gleichbleibender Topfzeit der erfindungsgemäßen Beschichtungen erkennen.

**Patentansprüche**

**1.** Zusammensetzung enthaltend einen oder mehrere Polyasparaginsäureester der allgemeinen Formel (I)

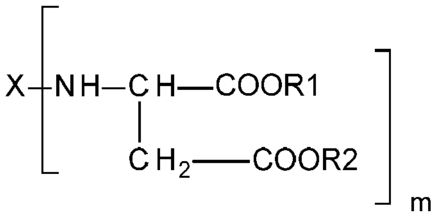

(I),

in welcher

X für einen m-wertigen, gegebenenfalls ein oder mehrere Heteroatome enthaltenden organischen Rest steht, wie er durch Entfernung der primären Aminogruppen aus einem entsprechenden, (cyclo)aliphatisch bzw. araliphatisch gebundene primäre Aminogruppen aufweisenden Polyamin des Molekulargewichtsbereichs 60 bis 6000 g/mol erhalten werden kann, und der weitere, gegenüber Isocyanatgruppen reaktive und/oder bei Temperaturen bis 100°C inerte funktionelle Gruppen enthalten kann,
R1 und R2 für gleiche oder verschiedene organische Reste, mit je 1 bis 18 Kohlenstoffatomen stehen,
m für eine ganze Zahl >1 steht,

und
einen oder mehrere Polyasparaginsäureester mit primärer Aminogruppe der allgemeinen Formel (II)

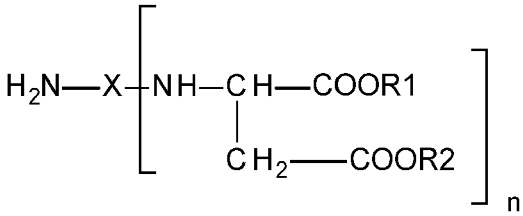

(II)

in welcher

n für m-1 steht,
X, Reste R1 und R2 die oben genannten Bedeutungen haben,

**dadurch gekennzeichnet, dass** der Anteil der Verbindung der allgemeinen Formel (II) von 1% bis 20% der GC Oberfläche (gemessen als Flächen-% im Gas Chromatogram), dem Anteil der beiden Verbindungen der allgemeinen Formel (I) und (II) entspricht, wobei die Summe der GC Oberflächen der beiden Verbindungen der allgemeinen Formel (I) und (II) 100% beträgt und ein Anteil an Fumarsäuredialkylester von 0.01 bis 3 Gew.-Prozent vorliegt.

**2.** Verfahren zur Herstellung der Zusammensetzung enthaltend ein oder mehrere Polyasparaginsäureester der allgemeinen Formel (I)

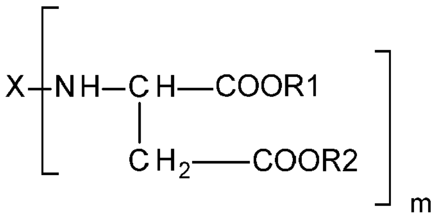

(I),

in welcher

X für einen m-wertigen, gegebenenfalls ein oder mehrere Heteroatome enthaltenden organischen Rest steht, wie er durch Entfernung der primären Aminogruppen aus einem entsprechenden, (cyclo)aliphatisch bzw. araliphatisch gebundene primäre Aminogruppen aufweisenden Polyamin des Molekulargewichtsbereichs 60 bis 6000 g/mol erhalten werden kann, und der weitere, gegenüber Isocyanatgruppen reaktive und/oder bei Temperaturen bis 100°C inerte funktionelle Gruppen enthalten kann,
R1 und R2 für gleiche oder verschiedene organische Reste, vorzugsweise für gleiche oder verschiedene Alkylreste mit je 1 bis 18 Kohlenstoffatomen und ganz besonders bevorzugt für gleiche oder verschiedene Alkylreste mit je 1 bis 8 Kohlenstoffatomen stehen,
m für eine ganze Zahl > 1 steht,

und
einen oder mehrere Polyasparaginsäureester mit primärer Aminogruppe der allgemeinen Formel (II)

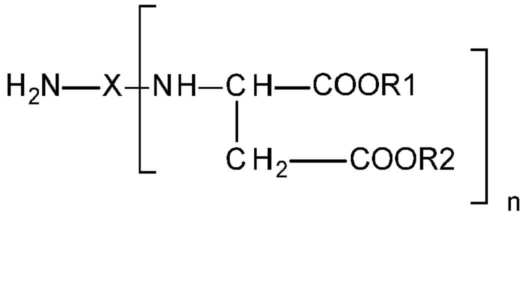

(II)

in welcher

n für m-1 steht,

X, Reste R1 und R2 die oben genannten Bedeutungen haben,

hergestellt durch Umsetzung von Polyaminen der allgemeinen Formel (III),

$$X\text{-}[NH_2]_m \qquad (III),$$

wobei X und m die oben genannte Bedeutung haben,(III), mit Verbindungen der allgemeinen Formel (IV)

$$R1OOC\text{-}CH{=}CH\text{-}COOR2 \qquad (IV),$$

wobei die Reste R1 und R2 die oben genannte Bedeutung haben, **dadurch gekennzeichnet, dass** die daraus resultierende Polyasparaginsäureester-Zusammensetzung einen Anteil der Verbindung der allgemeinen Formel (II) von 1% bis 20% der GC Oberfläche (gemessen als Flächen-% im Gas Chromatogram), dem Anteil der beiden Verbindungen der allgemeinen Formel (I) und (II) entspricht, wobei die Summe der GC Oberflächen der beiden Verbindungen der allgemeinen Formel (I) und (II) 100% beträgt und einen Anteil an Fumarsäuredialkylester von 0.01 bis 3 Gew.-Prozent enthält.

**3.** Verwendung der Polyasparaginsäureester-Zusammensetzungen gemäß Anspruch 1 als Reaktivkomponente in Zweikomponenten-Polyurethan-Systemen oder zur Herstellung von Prepolymeren.

**4.** Beschichtete Substrate, die unter Einsatz der Polyasparaginsäureester-Zusammensetzungen gemäß Anspruch 1 erhältlich sind.

Europäisches Patentamt
European Patent Office
Office européen des brevets

# EUROPÄISCHER RECHERCHENBERICHT

Nummer der Anmeldung
EP 17 19 1792

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2014/151307 A1 (BAYER MATERIALSCIENCE LLC [US]; SQUILLER EDWARD P [US]; WAYT TERRELL D) 25. September 2014 (2014-09-25) * Beispiel 1 * ----- | 1-4 | INV. C08G18/79 C07C229/24 C09D175/02 C08G18/38 |
| X,D | EP 0 816 326 A1 (BAYER AG [DE]) 7. Januar 1998 (1998-01-07) * Beispiele 6,7 * ----- | 1-4 | |
| X,D | EP 1 197 507 A2 (BAYER AG [DE]) 17. April 2002 (2002-04-17) * Beispiele 3,4 * ----- | 1-4 | |
| A,D | WO 2015/130502 A1 (AXALTA COATING SYSTEMS IP CO [US]; COATINGS FOREIGN IP CO LLC [US]) 3. September 2015 (2015-09-03) * Seite 29, Zeile 23 - Seite 30, Zeile 12 * ----- | 1-4 | |
| A,D | EP 0 403 921 A2 (BAYER AG [DE]) 27. Dezember 1990 (1990-12-27) * Seite 5, Zeilen 1-5 * ----- | 1-4 | RECHERCHIERTE SACHGEBIETE (IPC) |
| A | CN 101 469 246 A (SHANGHAI COATINGS CO LTD [CN]) 1. Juli 2009 (2009-07-01) * Beispiele 1-3 * ----- | 1-4 | C08G C07C C09D |

1

Der vorliegende Recherchenbericht wurde für alle Patentansprüche erstellt

| Recherchenort | Abschlußdatum der Recherche | Prüfer |
|---|---|---|
| München | 19. Februar 2018 | Lanz, Sandra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 17 19 1792

In diesem Anhang sind die Mitglieder der Patentfamilien der im obengenannten europäischen Recherchenbericht angeführten Patentdokumente angegeben. Die Angaben über die Familienmitglieder entsprechen dem Stand der Datei des Europäischen Patentamts am Diese Angaben dienen nur zur Unterrichtung und erfolgen ohne Gewähr.

19-02-2018

| Im Recherchenbericht angeführtes Patentdokument | Datum der Veröffentlichung | Mitglied(er) der Patentfamilie | Datum der Veröffentlichung |
|---|---|---|---|
| WO 2014151307 A1 | 25-09-2014 | CN 105073815 A | 18-11-2015 |
| | | EP 2970556 A1 | 20-01-2016 |
| | | US 2016024339 A1 | 28-01-2016 |
| | | WO 2014151307 A1 | 25-09-2014 |
| EP 0816326 A1 | 07-01-1998 | AT 192734 T | 15-05-2000 |
| | | CA 2209131 A1 | 02-01-1998 |
| | | EP 0816326 A1 | 07-01-1998 |
| | | ES 2148877 T3 | 16-10-2000 |
| | | JP 4173567 B2 | 29-10-2008 |
| | | JP H1087583 A | 07-04-1998 |
| | | US 5821326 A | 13-10-1998 |
| EP 1197507 A2 | 17-04-2002 | AT 296845 T | 15-06-2005 |
| | | CA 2358760 A1 | 11-04-2002 |
| | | DE 10050137 A1 | 18-04-2002 |
| | | EP 1197507 A2 | 17-04-2002 |
| | | ES 2242691 T3 | 16-11-2005 |
| | | JP 4355460 B2 | 04-11-2009 |
| | | JP 2002121178 A | 23-04-2002 |
| | | MX PA01010297 A | 10-11-2004 |
| | | PT 1197507 E | 30-09-2005 |
| | | US 2002132965 A1 | 19-09-2002 |
| WO 2015130502 A1 | 03-09-2015 | CN 106029729 A | 12-10-2016 |
| | | EP 3110869 A1 | 04-01-2017 |
| | | US 2017058146 A1 | 02-03-2017 |
| | | WO 2015130502 A1 | 03-09-2015 |
| EP 0403921 A2 | 27-12-1990 | AT 113622 T | 15-11-1994 |
| | | AU 623536 B2 | 14-05-1992 |
| | | BR 9002948 A | 20-08-1991 |
| | | CA 2018803 A1 | 23-12-1990 |
| | | DD 297982 A5 | 30-01-1992 |
| | | EP 0403921 A2 | 27-12-1990 |
| | | ES 2062188 T3 | 16-12-1994 |
| | | JP H0343472 A | 25-02-1991 |
| | | JP H0653871 B2 | 20-07-1994 |
| | | US 5126170 A | 30-06-1992 |
| CN 101469246 A | 01-07-2009 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0403921 A
- EP 0639628 A
- EP 0667362 A
- EP 0689881 A
- US 5214086 A
- EP 0699696 A
- EP 0596360 A
- EP 0893458 A
- DE 19701835
- US 5243012 A
- EP 0816326 A
- EP 1197507 A
- DE 102006002153
- WO 15130501 A
- WO 15130502 A

### In der Beschreibung aufgeführte Nicht-Patentliteratur

- **HAUBEN WEYL.** *Meth. d. Org. Chemie Bd.,* 1957, vol. 11/1, 272
- *Usp. Khim.,* 1969, vol. 38, 1933